# EUROPEAN PATENT SPECIFICATION

(11) **EP 2 035 029 B1**
(45) Date of publication and mention of the grant of the patent: **30.11.2016**
(21) Application number: 07764678.4
(22) Date of filing: 15.06.2007
(51) Int. Cl.: A61K 38/04, A61P 25/08, A61K 31/55

(54) **PEPTIDE COMPOUNDS FOR TREATING REFRACTORY STATUS EPILEPTICUS**
PEPTIDVERBINDUNGEN ZUR BEHANDLUNG VON REFRAKTÄREM STATUS EPILEPTICUS
COMPOSÉS PEPTIDIQUES DESTINÉS AU TRAITEMENT DE L'ÉTAT DE MAL ÉPILEPTIQUE RÉFRACTAIRE

(30) Priority: 15.06.2006 US 813967 P; 30.06.2006 EP 06013655
(43) Date of publication of application: 18.03.2009
(73) Proprietor: UCB Pharma GmbH, 40789 Monheim (DE)
(72) Inventor: STÖHR, Thomas, 40789 Monheim (DE)
(74) Representative: Weickmann & Weickmann PartmbB
(86) International application number: PCT/EP2007/005306
(87) International publication number: WO 2007/144196

(56) References cited:
- EP-A- 1 541 138
- US-A- 5 378 729
- STOEHR T ET AL: "The pre-clinical profile of the novel anticonvulsant lacosamide" EPILEPSIA, RAVEN PRESS LTD., NEW YORK, vol. 46, no. suppl. 6, 1 September 2005 (2005-09-01), pages 373-374, XP009092396 ISSN: 0013-9580
- CONLEY ET AL: "Functionalized DL-amino acid derivatives. Potent new agents for the treatment of epilepsy" JOURNAL OF MEDICINAL CHEMISTRY, AMERICAN CHEMICAL SOCIETY. WASHINGTON, vol. 30, no. 3, 1 January 1987 (1987-01-01), pages 567-574, XP002106737 ISSN: 0022-2623
- HORSTMANN R ET AL: "SPM 927 DOES NOT INTERACT WITH VALPROIC ACID AND CARBAMAZEPINE" MOLECULAR IMMUNOLOGY, ELMSFORD, NY, US, vol. 44, no. SUPPL. 09, 5 December 2003 (2003-12-05), page 97, XP009028152 ISSN: 0161-5890
- FOUNTAIN N B ET AL: "ABSENCE OF EFFECT OF ADJUNCTIVE SPM 927 ON CONCOMITANT AED PLASMA CONCENTRATIONS IN SUBJECTS WITH PARTIAL SEIZURES" MOLECULAR IMMUNOLOGY, ELMSFORD, NY, US, vol. 44, no. SUPPL. 09, 5 December 2003 (2003-12-05), page 96, XP009028153 ISSN: 0161-5890
- REYNOLDS E H ET AL: "SINGLE DRUG OR COMBINATION THERAPY OF EPILEPSY?" DRUGS, ADIS INTERNATIONAL LTD, vol. 21, no. 5, 1 January 1981 (1981-01-01), pages 374-382, XP009029147 ISSN: 0012-6667
- HOVINGA C A: "SPM-927" IDRUGS, CURRENT DRUGS LTD, GB, vol. 6, no. 5, 1 May 2003 (2003-05-01), pages 479-485, XP001180322 ISSN: 1369-7056

## Description

The present application claims the priority of the US provisional application US 60/813,967 of 15 June 2006. The present application also claims the priority of EP 06013 655.3 of 30 June 2006.

The present invention is directed to a class of peptide compounds for use in the treatment of refractory *status epilepticus (SE)* or a related condition.

In particular, the present invention is directed to a combination of a class of peptide compounds and a drug used in the treatment of SE, such as benzodiazepines, anticonvulsants, or barbiturates, in particular a benzodiazepine for use in the treatment of refractory *status epilepticus* or/and a condition related to refractory *status epilepticus,* such as epileptogenesis or epileptogenesis caused by refractory *status epilepticus.*

U.S. Patent No. 5,387,729 describes peptide compounds exhibiting central nervous system (CNS) activity and are useful in the treatment of epilepsy, nervous anxiety, psychosis and insomnia. EP 1 541 138 describes peptide compounds useful for treatment of *status epilepticus.* Stoehr et al., Epilepsia 46, Suppl. 6 (2005):373-374 describe Lacosamide (SPM927) belonging to a class of anticonvulsant agents, and describe experiments in rodent models of partial and generalised epilepsy designed to describe its anticonvulsant profile.. However, neither of these documents describes these compounds for use in the treatment of refractory *status epilepticus* or/and a related condition, such as *epileptogenesis* or *epileptogenesis* caused by refractory *status epilepticus.*

Seizures are the consequence of a paroxsymal brain dysfunction related to excessive neuronal activity that leads to an alteration of behaviour or consciousness. Epilepsy represents the recurrence of two or more unprovoked seizures and represents a chronic brain disease. About 0.5% of the population suffers epilepsy, and up to 10% of the population will suffer at least one seizure during their life-time.

There are two major types of seizures: partial or focal seizures, which originate in a location in the brain, but can spread in the course of the event; and generalized seizures, which can affect both hemispheres simultaneously. Partial seizures are manifested in multiple ways (confusion, automatic body movements, hallucinations, etc.) depending on the area of the brain that is affected, and if they spread in the brain can end up in a generalized tonic-clonic event (a convulsion). A complex partial seizure is a type of partial seizure originating in the temporal lobe and characterized by impairment of consciousness, often preceded by a hallucinatory aura. If a partial seizure spreads in the brain it can end up as a generalized seizure, for example a tonic-clonic convulsion There are several types of generalized seizures: convulsive (tonic-clonic, tonic, clonic, myoclonic) and non-convulsive (absences, atonic). Typically all kinds of seizures last up to a few minutes, generally less than five minutes. Convulsive seizures, particularly tonic-clonic events, typically result in impairment of consciousness.

*Status epilepticus* is currently defined as a seizure that lasts for 30 or more minutes, or a series of consecutive seizures that occur for 30 or more minutes during which the subject does not completely recover consciousness. Many clinicians and many recent major research articles, however, consider a patient to be in status if seizures last more than 5 minutes.

For purposes of the present application, SE will be understood to mean any epileptic event in which a generalized or partial seizure lasts longer than 5 minutes, or in which a series of generalized or partial seizures occur during a period longer than 5 minutes without full recovery of consciousness between seizures.

There are two main types of *status epilepticus:* generalized (convulsive and non-convulsive) and focal. The generalized convulsive status is the most severe type and is associated with high morbidity and mortality. *Status epilepticus* can occur in patients with prior epilepsy diagnosis. However, the onset of status is more frequent in subjects without previous epilepsy and is often related to a severe and acute brain disease (for example, an encephalitis or a stroke). In addition to these, a variety of conditions including hypoglycemia, hyperthermia, drug overdose and alcohol or drug withdrawal can be a cause of SE. Thus, anticonvulsant activity of a compound or combination, for example in models for or patients with complex partial seizures, is not necessarily predictive for activity against SE. SE is not only a life threatening disease but also causes neuronal cell loss and epileptogenesis.

*Status epilepticus* or related conditions represent an emergency and pharmacological treatment should preferably be carried out using intravenous medication. Drugs currently used for initial treatment include intravenous benzodiazepines (for example diazepam or lorazepam), anticonvulsants (for example phenytoin, fosphenytoin or valproic acid) and barbiturates (for example phenobarbital). Intravenous valproic acid has also been used. Rectal or intramuscular administration routes may also be used. Despite these first line treatments, over 40% of the subjects will not respond. Under these circumstances, pharmacological coma, induced for example by pentobarbital, thiopental, propofol, high dose of midazolam or other benzodiazepines is needed to treat status.

Recent population-based studies indicate that *status epilepticus* still carries an acute mortality of 27% in adults, and there is a general consensus that standard drugs used are unsatisfactory. While they work relatively well if given very early in the course of *status epilepticus,* they lose their efficacy quickly if seizures continue for more than half an hour. Barbiturates and other GABAergic drugs never become totally inactive, but can require such high doses that toxic side effects prevent a fully effective treatment. In animal models for instance, the potency of benzodiazepines can decrease 20 times within 30 min of self-sustaining *status epilepticus.* Other anticonvulsants, such as phenytoin, also lose potency, but more slowly.

Thus, early initiation of anticonvulsants is crucial in current treatment of *status epilepticus* and to prevent its long-term consequences, e.g. neuronal cell loss and epileptogenesis (for review, see Chen JW, Wasterlain CG, Lancet Neurol 2006, 5:246-56).

Optimal treatment of refractory SE and the prevention of its consequences as defined herein have not been established.

The prevalence of epilepsy following status epilepticus is three time higher than following a single 'normal' seizure indicating the status epilepticus is highly epileptogenic (for review see Chen and Wasterlain Lancet Neurology 2006). So far no drug has shown inhibition of epileptogenesis induced by status epilepticus in humans.

Most frequently used is e.g. administration of midazolam (see Claassen et al., Neurology 57 (2001), 1036-1042), propofol, or pentobarbital (Stecker et al., Epilepsia 39 (1998), 18-26). A meta-analysis based on a literature review by Claassen et al. (Epilepsia 43 (2002), 146-153) of the response of patients in refractory SE to treatment with midazolam, propofol or pentobarbital revealed low treatment failure but high incidence of hypotension for pentobarbital, a high number of breakthrough seizures for midazolam and similar high numbers of withdrawal seizures for all therapies. Thus, there is an unmet need for further therapy options of refractory SE.

While seizures are the common symptom for both epilepsy and *status epilepticus, status epilepticus* frequently occurs in subjects not suffering from epilepsy. A variety of other diseases such as stroke, brain trauma or encephalitis or a variety of conditions like hypoglycaemia, hyperthermia, drug overdose or alcohol or drug withdrawal can be the cause of *status epilepticus.* Thus, the anticonvulsant activity in models for or patients with complex partial seizures is not necessarily predictive for an activity against *status epilepticus.*

Current anti-epileptic drugs are believed to work through diverse mechanisms of action, including for example altering neuronal impulse propagation via interaction with voltage-gated sodium, calcium or potassium channels, or affecting neural transmission either by potentiating inhibitory GABA (gamma-aminobutyric acid) systems or by inhibition of excitatory glutamate systems.

(R)-2-acetamido-N-benzyl-3-methoxypropionamide (lacosamide, previously called SPM 927 or harkoseride) is a functionalized amino acid initially synthesized as an anticonvulsant. Lacosamide appears to be more potent and effective as compared to other clinically effective anticonvulsant drugs (phenytoin, carbamazepine) when it was evaluated in several anticonvulsant animal models.

Especially where the refractory nature of SE cannot be overcome by increasing the dose of a standard anti-epileptic drug because of the risk of unacceptable adverse side effects, the present composition can be advantageous.

The use of compounds of Formula (II) for treatment of refractory *status epilepticus* has not been reported. Thus, the present invention concerns the compounds of Formula (II) for use in the prevention, alleviation or/and treatment of refractory epileptic condition, particularly refractory *status epilepticus,* or/and a condition related to refractory *status epilepticus.*

The present invention is defined by the claims.

Furthermore there is described a method for treating refractory *status epilepticus* or/and a related condition in a subject, comprising administering to the subject at least one compound of Formula (II).

Long-term consequences of status epilepticus including refractory *status epilepticus* are neuronal damage e.g. cell loss in the hippocampus and epileptogenesis i.e. the occurrence of spontaneous seizures at several months to years following the first *status epilepticus* event.

"Condition related to *status epilepticus"* or "condition related to refractory *status epilepticus"* as used herein includes a condition caused by *status epilepticus,* for example epileptogenesis or neuronal cell loss. "Epileptogenesis" as used herein includes the development of epilepsy, such as chronic epilepsy, or an epileptic condition as described herein.

Surprisingly, it was found that lacosamide (50 mg/kg) administered 40 min after onset of self sustaining *status epilepticus* (SSSE) in perforant path stimulated rats effected a 40% reduction in seizure frequency and cumulative seizure duration. The percentage of rats developing chronic epilepsy following 6 months was reduced from 100% to 30%. Similarly the number of seizures per week after six months was reduced by 60%. 6 months after induction of SSSE 100% of vehicle treated animals developed spontaneous recurrent seizures with an average of 110 seizures per week. Following treatment with lacosamide (30-50 mg/kg) only 30% of rats developed spontaneous recurrent seizures and seizure frequency reduced by 60%. Literature data indicate that the self sustaining *status epilepticus* in this model is responsive to treatment with benzodiazepines or hydantoins (phenytoin and fosphenytoin) within the first 10 min of *status epilepticus* but later becomes refractory to those agents (Mazarati et al. 1999, Neurosci. Lett. 265:187-190).

In the rat lithium/pilocarpine model of SE, lacosamide treatment (50 mg/kg) 10 minutes after onset of *status epilepticus* still resulted in reduced motor seizure symptoms while standard anti-status drugs were completely ineffective. Although standard anti-status drugs e.g. benzodiazepines were ineffective, a combination of 50 mg/kg lacosamide administered 10 min after onset of *status epilepticus* and 20 mg/kg diazepam administered 15 min after onset of *status epilepticus* was surprisingly superior over lacosamide alone, since full seizures control was achieved in all rats by this combination treatment.

From these experimental findings, it is concluded that the compounds of the present invention, in particular lacosamide, or a combination of the compounds of the present invention, in particular of lacosamide, with a further drug used in the treatment of SE, such as benzodiazepines, anticon-vulsants or barbiturates, in particular a benzodiazepine such as diazepam, lorazepam, midazolam, clonazepam, clorazepate or/and clobazan are suitable for the treatment of a long-lasting *status epilepticus* which has become refractory or which is or becomes refractory in the course of its duration.

The compounds of the present invention of Formula (II) ,in particular lacosamide, are well tolerated, which is an advantage over other commonly used therapeutics for treatment of refractory epileptic conditions such as refractory *status epilepticus.*

Without being bound by theory, the mode of action of the compounds of Formula (II) differs from that of common antiepileptic drugs. Ion channels are not affected by the compounds of the present invention in a manner comparable to other known antiepileptic drugs, whereas GABA-induced currents are potentiated, but no direct interaction with any known GABA receptor subtype is observed. Glutamate induced currents are attenuated but the compounds do not directly interact with any know glutamate receptor subtype.

As used herein, "epileptic condition" refers to a disease state including *status epilepticus,* an epileptic seizure, a repetitive seizure or/and a seizure cluster.

As used herein, "refractory epileptic condition" refers to a disease state including *status epilepticus,* an epileptic seizure, a repetitive seizure or/and a seizure cluster which is at least partially resistant or substantially resistant against one or more drugs employed in the treatment of *status epileptics* or/and epilepsy. In particular, these drugs are different from the compounds of Formula II as defined herein. More particular, it refers to a disease state which is at least partially refractory or substantially refractory against at least one drug selected from benzodiazepines, barbiturates, and anticonvulsants different from the compounds of Formula II as defined herein, particularly selected from diazepam, lorazepam, midazolam, phenobarbital, carbamazepine, phenytoin, fosphenytoin, oxcarbazepine, lamotrigine, gabapentin, pregabalin, valproic acid, pentobarbital, thiopental, propofol and pharmaceutically acceptable salts thereof.

A refractory *status epilepticus* or a related condition in a particular patient may be present a priori, or may be caused by the duration of *status epilepticus.*

In certain embodiments of the present invention, a refractory epileptic condition comprises *status epilepticus,* an epileptic seizure, a repetitive seizure or/and a seizure cluster, which has become at least partially refractory due to its duration for at least about 10 min, at least about 15 min, at least about 20 min, at least about 30 min, at least about 45 min, or at least about 60 min, preferably at least about 30 min, at least about 45 min, or at least about 60 min.

In certain embodiments, SE treated by a composition of the present invention is initially responsive to treatment with one or more drugs employed in the treatment of status epilepticus or/and epilepsy as described herein, but becomes at least partially refractory when it lasts for at least about 10 minutes, for example at least about 15 minutes, at least about 20 minutes, at least about 30 minutes, at least about 45 minutes or at least about 60 minutes.

The compounds of the present invention, in particular lacosamide, may be used in a first line treatment of a refractory condition considered to be refractory due to the duration of the disease state as defined above. More particularly, the pharmaceutical composition of the present invention is suitable for a first line treatment of refractory *status epilepticus* or a related condition.

The compounds of the present invention may also be used in a second line treatment of a refractory condition, wherein therapy resistance has already become apparent in a preceding treatment, in particular in a treatment with benzodiazepines, barbiturates, and anticonvulsants different from the compounds of the present invention, in particular phenytoin, phosphenytoin, and valproate. More particularly, the pharmaceutical composition of the present invention is suitable for a second line treatment of refractory *status epilepticus* or a related condition.

The seizures in refractory *status epilepticus* may be focal seizures or/and may be generalized seizures. The generalized seizures may be convulsive generalized seizures, such as tonic-clonic, tonic, clonic, or myoclonic seizures, or may be non-convulsive seizures, such as absences or atonic seizures. Typically, the refractory *status epilepticus* involves at least partial loss of conciousness.

Thus, in one embodiment, the refractory *status epilepticus* or a related condition comprises focal seizures or/and generalized seizures. In another embodiment, the refractory *status epilepticus* or a related condition comprises convulsive seizures, such as tonic-clonic, tonic, clonic, or myoclonic seizures, or/and non-convulsive seizures, such as absences or atonic seizures.

In yet another embodiment, the refractory *status epilepticus* or a related condition comprises acute repetitive seizures or/and seizure clusters.

Yet another aspect of the present invention is a pharmaceutical composition comprising at least one compound of Formula II as defined herein, preferably lacosamide, for use in the prevention, alleviation or/and treatment of refractory epileptic condition such as refractory *status epilepticus* or a related condition.

As discussed above, a combination of lacosamide and diazepam administered 15 min after onset of *status epilepticus* was surprisingly found to be superior to lacosamide alone in an animal model of status *epilepticus,* since full seizures control was achieved in all rats by this combination treatment. Diazepam, alone administered at this point in time was found to be ineffective. Thus, the compounds of Formula II may also be administered together with a further active agent, e.g. an antiepileptic drug, particularly a benzodiazepine drug.

A further aspect of the present invention is a pharmaceutical composition comprising
(a) at least one compound of Formula II as defined herein, preferably lacosamide, and
(b) at least one further active agent, e.g. a benzodiazepine, preferably diazepam, lorazepam, midazolam, clonazepam, clorazepate or/and clobazan.

In a particular embodiment, the further active agent is an anti-epileptic agent, for example comprising at least one benzodiazepine, barbiturate, and/or anticonvulsive other than a compound of Formula (II).

In one embodiment a pharmaceutical composition comprises one of the specific combinations lacosamide and diazepam, lacosamide and lorazepam, or lacosamide and midazolam. In this embodiment, the compound of (a) is lacosamide and the compound of (b) is diazepam, lorazepam or midazolam.

As used herein, "benzodiazepine" includes any benzodiazepine employed for treatment of *status epilepticus,* including diazepam, lorazepam, midazolam, clonazepam, clorazepate and clobazan. Preferred benzodiazepines are diazepam, lorazepam, or/and midazolam. Further antiepileptic drugs also include anticonvulsants or/and barbiturates.

The pharmaceutical composition comprising the combination of agents (a) and (b) as defined above is beneficial for the prevention, alleviation or/and treatment of any epileptic condition, particularly a refractory condition as defined above.

Refractory epileptic conditions treatable by the composition of the present embodiment include not only refractory SE as defined above, but also epileptic seizures, repetitive seizures and seizure clusters that are at least partially resistant or substantially resistant to treatment with anti-epileptic drugs such as benzodiazepines, barbiturates and/or anticonvulsants other than compounds of Formula (II), including seizures that do not necessarily involve loss of consciousness.

Yet another aspect of the present invention is at least one compound of Formula II as defined herein, in particular lacosamide, for use in the prevention, alleviation or/and treatment of epileptogenesis. In this use, "epileptogenesis" includes all embodiments of epileptogenesis as described herein.

Yet another aspect described herein is a method for the prevention, alleviation or/and treatment of epileptogenesis comprising administering to a subject in need thereof at least one compound of Formula II as defined herein, in particular lacosamide. In this method, "epileptogenesis" includes all aspects of epileptogenesis as described herein. In one aspect, epileptogenesis is related to *status epilepticus,* such as refractory *status epilepticus.*

In one aspect, the method for the prevention, alleviation or/and treatment of epileptogenesis of the present invention comprises administering a further active agent, particularly a benzodiazepine such as diazepam, lorazepam, or/and midazolam.

In another aspect, in the method for the prevention, alleviation or/and treatment of epileptogenesis described herein, the at least one compound of Formula II as defined herein, in particular lacosamide, is administered after onset of *status epilepticus.* In the method for the prevention, alleviation or/and treatment of epileptogenesis described herein, the at least one compound of Formula II may be administered at least about 10 minutes or at least 30 min after onset of *status epilepticus.* The compound of Formula II in particular lacosamide, may be administered at a dose of about 50 to about 500 mg. The compound of Formula II, in particular lacosamide, may be administered intravenously.

The compound of Formula II and the further active agent, e.g. the benzodiazepine may be formulated in one pharmaceutical preparation (single dosage form) for administration at the same time or may be formulated in two or more distinct preparations (separate dosage forms) for simultaneous or/and subsequent administration. The two distinct preparations in the separate dosage forms may be administered by the same route or by different routes.

Separate dosage forms can optionally be co-packaged, for example in a single container or in a plurality of containers within a single outer package, or co-presented in separate packaging ("common presentation,"). As an example of co-packaging or common presentation, a kit is contemplated comprising, in separate containers, compound of Formula II and the benzodiazepine. In another example, the compound of Formula II
and the further active agent, e.g. the benzodiazepine are separately packaged and available for sale independently of one another, but are co-marketed or co-promoted for use according to the invention. The separate dose forms may also be presented to a subject separately and independently, for use according to the invention.

In a further embodiment, the pharmaceutical composition comprises a single dosage form comprising at least one compound of Formula II and at least one further active agent, e.g. a benzodiazepine.

There is still further provided a pharmaceutical composition comprising at least one compound of Formula II at least one benzodiazepine, and at least one pharmaceutically acceptable excipient.

In another embodiment, the pharmaceutical composition of the present invention comprises separate dosage forms comprising
(i) a first composition comprising at least one compound of Formula II, and
(ii) a second composition comprising at least one further active agent, e.g. a benzodiazepine.

In yet another embodiment of the present invention, the second composition (ii) comprising the at least one further active agent may be a commercially available composition.

The pharmaceutical composition of the present invention is in one embodiment prepared for administration in mammals, preferably in humans.

The pharmaceutical composition of the present invention, in particular the composition comprising at least one compound of the present invention and at least one benzodiazepine may be prepared for administration at least about 10 min, at least about 15 min, at least about 20 min, at least about 40 min, at least about 45 min, or at least about 60 min, preferably at least about 30 min, at least about 45 min, or at least about 60 min after the onset of a *status epilepticus* or a related condition.

The pharmaceutical composition of the present invention, in particular the composition comprising at least one compound of the present invention and at least one benzodiazepine may be prepared for administration to patients suffering from refractory *status epilepticus,* which is at least partially or substantially resistant against drugs employed in the treatment of *status epilepticus,* particularly selected from diazepam, lorazepam, midazolam, phenobarbital, carbamazepine, phenytoin, fosphenytoin, oxcarbazepine, lamotrigine, gabapentin, pregabalin, valproic acid, pentobarbital, thiopental, propofol and pharmaceutically acceptable salts thereof.

The pharmaceutical composition of the present invention comprising (a) at least one compound of Formula (II) and (b) at least one further active agent, e.g. a benzodiazepine may be prepared for the prevention, alleviation or/and treatment of a *status epilepticus* (including refractory and non-refractory *status epilepticus) or*/*and* epilepsy.

Yet another aspect described herein is a method for the prevention, alleviation or/and treatment of a refractory epileptic condition, wherein the method comprises administering to a subject in need thereof at least one compound of Formula II, in particular lacosamide, optionally together with a further active agent, e.g. a benzodiazepine.

In one embodiment, the method further comprises administering a second active agent, in particular an anti-epileptic agent selected from benzodiazepines, barbiturates and anticonvulsive agents other than a compound of Formula (II).

A further aspect described herein is a method for the prevention, alleviation or/and treatment of an epileptic condition, wherein the method comprises co-administering to a subject in need thereof at least one compound of Formula II in particular lacosamide, and a benzodiazepine, in particular diazepam, lorazepam, or/and midazolam, in therapeutically effective amounts. One aspect comprises the co-administration of one of the specific combinations lacosamide and diazepam, lacosamide and lorazepam, or lacosamide and midazolam.

According to any of the above embodiments, an illustrative compound of Formula (II) is lacosamide, (R)-2-acetamido-N-benzyl-3-methoxypropionamide.

In the method of-the present invention, the at least one compound of the present invention, alone or in combination with at least one further compound, e.g. a benzodiazepine, is preferably administered to a subject in need thereof after the onset of the condition, e.g. about 10 min, about 15 min, about 20 min, about 30 min, about 40 min, about 45 min, about 60 min or more after the onset of the condition.

The term "co-administration" refers to a plurality of agents that, when administered to a subject together or separately, are co-active in bringing therapeutic benefit to the subject. Such co-administration is also referred to as "combination", "combination therapy," "co-therapy," "adjunctive therapy" or "add-on therapy." For example, one agent can potentiate or enhance the therapeutic effect of another, or reduce an adverse side effect of another, or one or more agents can be effectively administered at a lower dose than when used alone, or can provide greater therapeutic benefit than when used alone, or can complementarily address different aspects, symptoms or etiological factors of a disease or condition.

Co-administration comprises administration of the combination of the agents in amounts sufficient to achieve or/and maintain therapeutically effective concentrations, e.g. plasma concentrations, in the subject in need thereof Co-administration comprises simultaneous or/and subsequent administration. Simultaneous administration comprises administration of the agents as a single or as different compositions.

Sequential administration normally comprises administration of the compound of Formula (II), for example lacosamide, and the second active agent within an interval of up to about 90 minutes, for example up to about 60, up to about 45, up to about 40, up to about 30, up to about 20, up to about 10 or up to about 5 minutes. The administration interval can depend on the dosage forms and routes of administration of the agents. The compound of Formula (II) for example lacosamide, may be administered first, or the second active agent may be administered first.

When the method further comprises administration of a second active agent, as described herein, the compound of Formula (II), for example lacosamide, and the second active agent, e.g., a benzodiazepine, may be formulated in one pharmaceutical preparation (single dosage form) for administration at the same time, or alternatively may be formulated in two or more distinct preparations (separate dosage forms) for simultaneous and/or sequential administration. Separate dosage forms may be administered by the same route or by different routes.

Separate dosage forms can optionally be co-packaged, for example in a single container or in a plurality of containers within a single outer package, or co-presented in separate packaging ("common presentation"). As an example of co-packaging or common presentation, a kit is contemplated comprising, in separate containers, a compound of Formula (II) and a benzodiazepine. In another example, a compound of Formula (II) and a benzodiazepine are separately packaged and available for sale independently of one another, but are co-marketed or co-promoted for use according to the invention. Separate dosage forms may also be presented to a subject separately and independently, for use according to the invention.

In yet another embodiment of the present invention, a therapeutic combination comprises at least one compound of Formula (II) for example lacosamide, and at least one benzodiazepine. The combination can be used for treatment of any medical condition responsive thereto, including without limitation epileptic conditions such as SE, for example where such conditions are or become refractory as described above.

Any benzodiazepine can be used in the combination, particularly a benzodiazepine having anti-epileptic activity such as one or more of diazepam, lorazepam, midazolam, clonazepam, clorazepate and clobazan.

The compound of Formula (II) for example lacosamide, and the benzodiazepines, for example diazepam, lorazepam or midazolam, are present in the combination in therapeutically effective total and relative amounts. For example, in a combination comprising lacosamide and diazepam, lacosamide can be present in an amount providing a dose of about 50 to about 500 mg and diazepam in an amount providing a dose of about 10 to about 100 mg.

Each of the components of the therapeutic combination can be provided in a pharmaceutical composition adapted for the desired route of delivery, for example as an injectable composition where the components are to be administered intravenously. Each pharmaceutical composition comprises one or more excipient ingredients as more fully described above. The benzodiazepine, for example, can be provide in the form of a commercially available pharmaceutical composition.

Alternatively, the compound of Formula (II), for example lacosamide, and the benzodiazepine can be provided in a single pharmaceutical composition adapted for a particular route of administration.

Accordingly, in yet another embodiment of the present invention, a pharmaceutical composition comprises
(a) at least one compound of Formula (II), for example lacosamide:
(b) at least one benzodiazepine, for example diazepam, lorazepam, midazolam, clonazepam, clorazepate and/or clobazan; and
(c) at least one pharmaceutically acceptable excipient.

As used herein, "pharmaceutically acceptable excipients" includes any and all such materials mentioned above, and any and all solvents, dispersion media, coatings, antibacterial and antifungal agents, isotonic and absorption delaying agents for pharmaceutically active substances known in the art. Except insofar as any conventional excipient is incompatible with one or both active ingredients, its use in a pharmaceutical composition of the present embodiment is contemplated. One or more further active agents, in addition to those specified above, can optionally be present..

Since *status epilepticus* is an emergency situation, immediate administration of the compound of Formula II and optionally a further active agent, e.g. a benzodiazepine is required. Subsequent administration comprises administration of the compound of Formula II and the further active agent within an interval of up to 5 min, up to 10 min, up to 20 min, up to 30 min, up to 40 min, up to 60 min, or up to 90 min. The administration interval of the compound of Formula II and the further active agent may depend on the dosage forms. The compound of Formula II may be administered first, or the further active agent may be administered first.

The compound of the present invention has the general Formula (II), 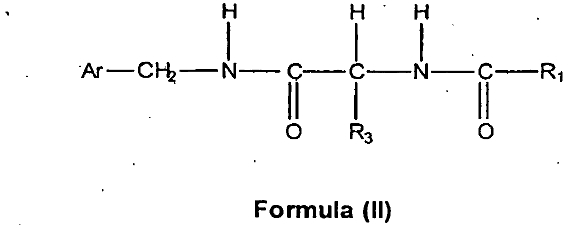 wherein
Ar is phenyl, which is unsubstituted or substituted with at least one halo group;
R₁ is alkyl containing 1-3 carbon atoms, preferably methyl; and
R₃ is -CH₂-Q, wherein Q is alkoxy containing 1 to 3 carbon atoms; or a pharmaceutically acceptable salt thereof.

The compounds utilized in the present invention may contain one or more asymmetric carbons and may exist in racemic and optically active forms. The configuration around each asymmetric carbon can be either the D or L form. It is well known in the art that the configuration around a chiral carbon atoms can also be described as R or S in the Cahn-Prelog-Ingold nomenclature system. All of the various configurations around each asymmetric carbon, including the various enantiomers and diastereomers as well as racemic mixtures and mixtures of enantiomers, diastereomers or both are contemplated by the present invention.

As used herein, the term configuration particularly refers to the configuration around the carbon atom to which H and R₃ are attached, even though other chiral centers may be present in the molecule. Therefore, when referring to a particular configuration, such as D or L, it is to be understood to mean the D or L stereoisomer at the carbon atom to which H and R₃ are attached. However, it also includes all possible enantiomers and diastereomers at other chiral centers, if any, present in the compound.

The compounds of the present invention are directed to all the optical isomers, i.e., the compounds of the present invention are either the L-stereoisomer or the D-stereoisomer (at the carbon atom to which H and R₃ are attached). These stereoisomers may be found in mixtures of the L and D stereoisomer, e.g., racemic mixtures. The D stereoisomer is preferred.

It is preferred that the compounds of Formula (II) are in the R configuration configuration.

It is preferred that the compounds of Formula (II) in the R configuration are substantially enantiopure. As used herein, the term "substantially enantiopure" refers to a content of the R enantiomer of at least 99.5%. This corresponds to an enantiomeric excess (ee) of 99%. The respective quantities of R and S enantiomer may be determined by chiral column chromatography, e.g. by HPLC with "ChiralPak" as chiral, stationary phase.

In one embodiment the compound, for example lacosamide, is substantially enantiopure.

As used herein, the term "substantially enantiopure" means having at least 88%, preferably at 90%, more preferably at least 95, 96, 97, 98, or 99% enantiomeric purity.

The term "alkyl" (alone or in combination with another term(s)) means a straight- or branched-chain saturated hydrocarbyl substituent preferably containing from 1 to about 20 carbon atoms (C₁-C₂₀-alkyl), more preferably from 1 to about 8 carbon atoms (C₁-C₈-alkyl), even more preferably from 1 to about 6 carbon atoms (C₁-C₅-alkyl), and most preferably from 1 to 3 carbon atoms (C₁-C₃-alkyl). The alkyl groups include methyl, ethyl, propyl, isopropyl, butyl, isobutyl, tertiary butyl, amyl, hexyl, and the like. Further, alkyl groups also include halogenated alkyl groups up to perhalogenation, e.g. trifluoromethyl, if not indicated otherwise.

The term "alkoxy" (alone or in combination with another term(s)) refers to -O-alkyl and means a straight- or branched-chain alkoxy substituent preferably containing from 1 to about 20 carbon atoms (C₁-C₂₀)-alkoxy), more preferably from 1 to about 8 carbon atoms (C₁-C₈-alkoxy), even more preferably from 1 to about 6 carbon atoms (C₁-C₆-alkoxy), and most preferably from 1 to 3 carbon atoms (C₁-C₃-alkoxy). The alkoxy groups include methoxy, ethoxy, propoxy, butoxy, isobutoxy, tert-butoxy, pentoxy, hexoxy and the like. Further, alkoxy groups include halogenated alkoxy groups up to perhalogenation, if not indicated otherwise.

The term "halo" or "halogen" includes fluoro, chloro, bromo, and iodo.

The prefix "halo" indicates that the substituent to which the prefix is attached is substituted with one or more independently selected halogen radicals. For example, haloalkyl means an alkyl substituent wherein at least one hydrogen radical is replaced with a halogen radical. Examples of haloalkyls include chloromethyl, 1-bromoethyl, fluoromethyl, difluoromethyl, trifluoromethyl, 1,1,1-trifluoroethyl, and the like. Illustrating further, "haloalkoxy" means an alkoxy substituent wherein at least one hydrogen radical is replaced by a halogen radical. Examples of haloalkoxy substituents include chloromethoxy, 1-bromoethoxy, fluoromethoxy, difluoromethoxy, trifluoromethoxy (also known as "perfluoromethyloxy"), 1,1,1,-trifluoroethoxy, and the like. It should be recognized that if a substituent is substituted by more than one halogen radical, those halogen radicals may be identical or different (unless otherwise stated).

Preferably Q is ethoxy or methoxy.

In the compounds of Formula (II), Ar is phenyl unsubstituted or substituted with at least one halo, preferably with at least one fluoro. More preferably Ar in Formula (II) is unsubstituted phenyl.

The most preferred compounds of the present invention include:
(R)-2-acetamido-N-benzyl-3-methoxy-propionamide;
(R)-2-acetamido-N-benzyl-3-ethoxy-propionamide;
O-methyl-N-acetyl-D-serine-m-fluorobenzyl-amide; and
O-methyl-N-acetyl-D-serine-p-fluorobenzyl-amide.

Other compounds described herein are:
N-acetyl-D-phenylglycine benzylamide;
D-1,2-(N,O-dimethylhydroxylamino)-2-acetamide acetic acid benzylamide;
D-1,2-(O-methylhydroxylamino)-2-acetamido acetic acid benzylamide;
D-α-acetamido-N-(2-fluorobenzyl)-2-furanacetamide; and
D-α-acetamido-N-(3-fluorobenzyl)-2-furanacetamide.

It is to be understood that the various combinations and permutations of the Markush groups of R₁ and R₃ described herein are contemplated to be within the scope of the present invention. Moreover, the present invention also encompasses compounds and compositions which contain one or more elements of each of the Markush groupings in R₁ and R₃ and the various combinations thereof. Thus, for example, the present invention contemplates that R₁ may be one or more of the substituents listed hereinabove in combination with any and all of the substituents of R₃.

More preferred is a compound of Formula (II) in the R configuration, preferably substantially enantiopure, wherein the substituent R is benzyl which is unsubstituted with at least one halo group, wherein R₃ is CH₂-Q, wherein Q is alkoxy containing 1-3 carbon atoms and wherein R₁ is methyl. Preferably R is unsubstituted benzyl or benzyl substituted with at least one halo group which is a fluoro group.

Depending upon the substituents, the compounds may form addition salts as well. All of these forms are contemplated to be within the scope of this invention including mixtures of the stereoisomeric forms.

The manufacture of compounds utilized in the present invention is described in U.S. Patent Nos. 5,378,729 and 5,773,475, and in the international application PCT/EP 2005/010603.

The compounds utilized in the present invention are useful as such as depicted in the Formula (II) or can be employed in the form of salts in view of its basic nature by the presence of the free amino group. Thus, the compounds of Formula (II) form salts with a wide variety of acids, inorganic and organic, including pharmaceutically acceptable acids. The salts with therapeutically acceptable acids are of course useful in the preparation of formulation where enhanced water solubility is most advantageous.

These pharmaceutically acceptable salts have also therapeutic efficacy. These salts include salts of inorganic acids such as hydrochloric, hydroiodic, hydrobromic, phosphoric_{,} metaphosphoric, nitric acid and sulfuric acids as well as salts of organic acids, such as tartaric, acetic, citric, malic, benzoic, perchloric, glycolic, gluconic, succinic, aryl sulfonic, (e.g., p-toluene sulfonic acids, benzenesulfonic), phosphoric, malonic, and the like.

The SE treated by a method described herein is at least partially refractory or substantially refractory against at least one anti-epileptic drug, for example a benzodiazepine, barbiturate or anticonvulsive other than a compound of Formula (II). In a particular embodiment, the at least one anti-epileptic drug to which the SE is refractory is selected from the group consisting of diazepam, lorazepam, midazolam, phenobarbital, carbamazepine, phenytoin, fosphenytoin, oxcarbazepine, lamotrigine, gabapentin, pregabalin, valproic acid, pentobarbital, thiopental, propofol and pharmaceutically acceptable salts thereof.

A compound of Formula (II), for example lacosamide, can be used in a therapeutically effective amount.

The physician will determine the dosage of the present therapeutic agents which will be most suitable and it will vary with the form of administration and the particular compound chosen, and furthermore, it will vary with the patient under treatment, the age of the patient, the type of malady being treated. He will generally wish to initiate treatment with small dosages substantially less than the optimum dose of the compound and increase the dosage by small increments until the optimum effect under the circumstances is reached. When the composition is administered orally, larger quantities of the active agent will be required to produce the same effect as a smaller quantity given parenterally. The compounds are useful in the same manner as comparable therapeutic agents and the dosage.level is of the same order of magnitude as.is generally employed with these other therapeutic agents.

In one embodiment, the compounds of the present invention are administered in amounts ranging from about 1 mg to about 100 mg per kilogram of body weight per day, more preferably in amounts ranging from about 1 mg to about 10 mg per kilogram of body weight per day. This dosage regimen may be adjusted by the physician to provide the optimum therapeutic response. Patients in need thereof may be treated with doses of the compound of the present invention of at least 50 mg/day, preferably of at least 200 mg/day, more preferably of at least 300 mg/day, still more preferably of at least 400 mg/day and most preferably of at least 600 mg/day. Generally, a patient in need thereof may be treated with doses at a maximum of 6 g/day, more preferably a maximum of 1 g/day, still more preferably a maximum of 600 mg/day, and most preferably a maximum of 800 mg/day. In some cases, however, higher or lower doses may be needed.

In another preferred embodiment, the daily doses are increased until a predetermined daily dose is reached which is maintained during the further treatment.

Doses expressed herein on a daily basis, for example in mg/day, are not to be interpreted as requiring a once-a-day frequency of administration. For example, a dose of 300 mg/day can be given as 100 mg three times a day, or as 600 mg every second day.

More typically, in an emergency situation, a compound of Formula (II)
for example lacosamide, is administered not on a daily basis but *pro re nata* (p.r.n.), typically after onset of SE. A typical single dose of lacosamide, for example, is an amount of about 50 to about 500 mg. Such administration can occur, for example, at any time from immediately after onset until about 60 minutes after onset or even later. In various embodiments administration occurs about 10, about 15, about 20, about 30, about 45 or about 60 minutes after onset.

Refractory SE, especially where the SE is of the generalized convulsive type, is an emergency situation and it is generally important to administer medication as soon as possible after onset. Thus in a particular embodiment a compound of Formula (II) for example lacosamide, is administered immediately after onset of SE or as soon as possible the-reafter.

A compound of Formula (II), for example lacosamide, can be used in first line treatment of refractory SE, for example where prior SE episodes have proven refractory to other treatments.

Alternatively, a compound of Formula (II), for example lacosami-de; can be used in second line treatment of refractory SE, wherein resistance has already become apparent following a preceding first line treatment, such as with one or more benzodiazepines, barbiturates or anticonvulsants other than compounds of Formula (II), in particular phenytoin, fosphenytoin or valproic acid.

Typically in second line treatment, a compound of Formula (II) is administered at least about 10 minutes, for example at least about 15, at least about 20, at least about 30, at least about 45 or at least about 60 minutes, after onset of SE. This administration can occur independently of the time when a seizure or seizure cluster becomes refractory to a first line treatment, but in one embodiment occurs immediately or as soon as possible after resistance becomes apparent to the first line treatment.

In yet another embodiment, several divided doses may be administered daily. For example, three doses per day may be administered, preferably two doses per day. It is more preferred to administer a single dose per day.

In yet another preferred embodiment, an amount of the compounds of the present invention may be administered which results in a plasma concentration of 0.1 to 15 µg/ml (trough) and 5 to 18.5 µg/ml (peak), calculated as an average over a plurality of treated subjects, intravenous administration in emergency treatment might result in peak plasmid levels of up to 30 µg/ml.

The compounds of Formula (II) may be administered in a convenient manner, such as by oral, intravenous (where water soluble), intramuscular, intrathecal, rectal (e.g. suppository, gel, liquid, etc.) or subcutaneous routes. Oral, rectal or/and intravenous (i.v.) administration is preferred. In emergency treatment, i.v. administration is most preferred.

The pharmaceutical composition of the present invention may be prepared for the treatment regimen as described above, in particular for the treatment with doses as described above, to effect plasma concentrations as described above, for administration periods or/and administration routes as specified in the embodiments of the present invention as described above.

The compounds of Formula (II) may be orally administered, for example, with an inert diluent or with an assimilable edible carrier, or it may be enclosed in hard or soft shell gelatin capsules, or it may be compressed into tablets, or it may be incorporated directly into the food of the diet. For oral therapeutic administration, the active compound of Formula
(II) may be incorporated with excipients and used in the form of ingestible tablets, buccal tablets, troches, capsules, elixirs, suspensions, syrups, wafers, and the like. Such compositions and preparations should contain at least 1 % of active compound of Formula (II). The percentage of the compositions and preparations may, of course, be varied and may conveniently be between about 5 to about 80 % of the weight of the unit. The amount of active compound of Formula (II) in such therapeutically useful compositions is such that a suitable dosage will be obtrained. Preferred compositions or preparations according to the present invention contains between about 10 mg and 6 g active compound of Formula (II) for example about 50 to about 1000 mg, or about 100 to about 800 mg, of the compound.

The tabtets, troches, pills,' capsules and the like may also contain the following: A binder such as gum tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier.

Various other materials may be present as coatings or otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations. For example, sustained release dosage forms are contemplated wherein the active ingredient is bound to an ion exchange resin which, optionally, can be coated with a diffusion barrier coating to modify the release properties of the resin.

The active compound may also be administered parenterally or intraperitoneally. Dispersions can also be prepared in glycerol, liquid, polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersions and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of preparing sterile powders for the manufacture of sterile injectable solutions, the preferred methods of preparation are vacuum drying, or freeze-drying optionally together with any additional desired ingredient.

As used herein, "pharmaceutically acceptable carrier" includes any and all solvents, dispersion media, coatings, antibacterial and antifungal agent, isotonic and absorption delaying agents for pharmaceutical active substances as well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form or ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specifics for the novel dosage unit forms of the invention are dictated by and directly dependent on (a) the unique characteristics of the active material an the particular therapeutic effect to be achieved, and (b) the limitations inherent in the art of compounding such as active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore described. A unit dosage form can, for example, contain the principal active compound in amounts ranging from about 10 mg to about 6 g. Expressed in proportions, the active compound is generally present in from about 1 to about 750 mg/ml of carrier. In the case of compositions containing supplementary active ingredients, the dosages are determined by reference to the usual dose and manner of administration of the said ingredients.

As used herein the term "patient" or "subject" refers to a warm blooded animal, and preferably mammals, such as, for example, cats, dogs, horses, cows, pigs, mice, rats and primates, including humans. The preferred patient is a human.

The term "treat" refers to either relieving the pain associated with a disease or condition, to providing partial to complete relieve of the patient's disease or condition, or alleviating the patient's disease or condition. More specifically, unless the context demands otherwise, the term "treat," "treating" or "treatment" herein includes preventive or prophylactic use of a medication in a subject at risk of, or having a prognosis including, a refractory epileptic condition, as well as use of such a compound in a subject already experiencing a refractory epileptic condition, as a therapy to alleviate, relieve, reduce intensity of or eliminate such a condition or an underlying cause thereof. In a particular aspect, administration of a medication according to a method of the invention is post-onset of SE. At the time of administration the SE may already be refractory or, based on prior episodes or on the duration of the seizures, may have a prognosis of becoming refractory.

The compounds of the present invention are administered to a patient suffering from the aforementioned type of disorder in an effective amount. These amounts are equivalent to the therapeutically effective amounts described hereinabove.

The invention is further illustrated by the following figure and example.

### Figure Legends

**Figure 1****:** The effects of lacosamide in the self-sustained *status epilepticus* model for treatment-resistant *status epilepticus.*
**Figure 2****:** Effect of early treatment on number of SRS/week.
**Figure 3****:** Effect of late treatment on number of seizures/week.

### Examples

While standard anti-epileptic drugs can work relatively well if given very early in the course of SE, they typically lose their efficacy as seizures continue, especially if seizures continue for more than about 30 minutes.

These clinical features can be reproduced experimentally, using the perforant path stimulation model and the lithium/pilocarpine model of *status epilepticus.* Lacosamide was studied in these two models by administration at a defined time after onset of experimentally induced SE, at which time the standard drugs have a reduced efficacy or are even inactive. For example, Mazarati et al. (1999, Neurosci Lett. 265:187-190) stated that during the course of self-sustaining *status epilepticus* (SSSE) in the perforant paths stimulation (PPS) model, resistance to standard anticonvulsants developed progressively: diazepam and phenytoin were highly effective when given before or a onset of SSSE, but lost their effectiveness when administration was delayed.

Lacosamide was studied in the perforant path stimulation model and the lithium/pilocarpin model of status epilepticus. Lacosamide was studied in these two models for treatment of refractory status epilepticus, wherein lacosamide is administered at a defined period after onset of the experimentally induced *status epilepticus,* at which time the standard drugs have a reduced efficacy or are even inactive.

The following examples illustrate anticonvulsive efficacy of lacosamide, alone and in combination with diazepam, in models for refractory SE.

### Example 1: Perforant path stimulation model

Male Wistar rats were implanted with a stimulating electrode into the angular bundle of the perforant path and a recording electrode into the granule cell layer of the dentate gyrus. Perforant path stimulation (PPS) was delivered for 30 or 60 minutes with the following parameters: 10 s, 20 Hz trains of 1 ms, 30 V pulses delivered every minute together with continuous 2 Hz stimulation with the same parameters.

Lacosamide was injected intraperitoneally 40 minutes after the end of PPS at a dose of 50 mg/kg. The following indices were used to quantify seizure activity: cumulative seizure time (duration of SSSE, subtracting interictal time) and the number of seizure episodes. In addition the number of spontaneous seizures was measured 6 months following induction of SSSE in order to assess status epilepticus induced epileptogenesis.

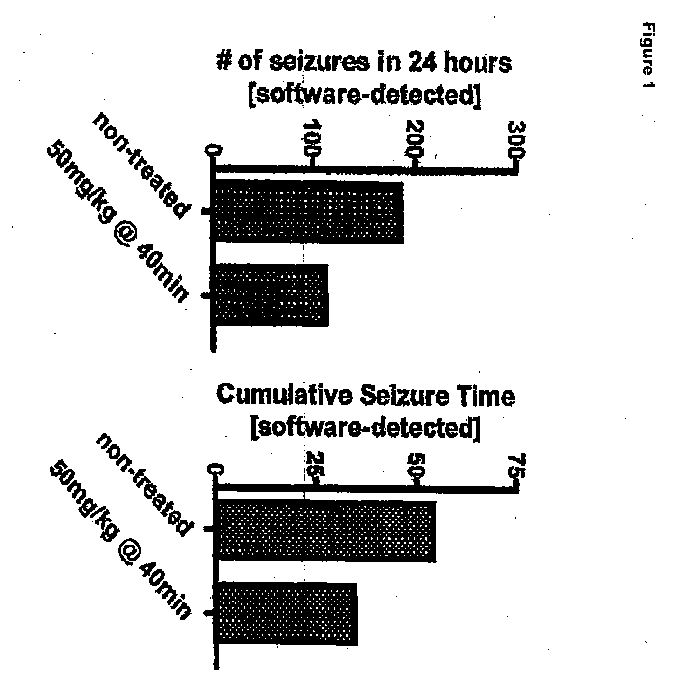

When lacosamide treatment was initiated 40 minutes after PPS, a substantial reduction in both seizure frequency and cumulative seizure duration was obtained, as shown in Fig. 1.

### Example 2: Lithium/pilocarpine model

Rats received 3 mmol/kg lithium 20-24 hours prior to administration of 40 mg/kg pilocarpine. Lacosamide treatment was initiated after 10 minutes of high amplitude rapid continuous spiking on EEG. This is a time that has previously been demonstrated to be refractory to treatment with standard clinical anti-SE drugs in this model (see, for example, a study of response to diazepam by Walton & Treiman (1988) Exp. Neurol. 101:267-275).

Treatment with lacosamide (50 mg/kg) reduced motor seizure symptoms under conditions where standard anti-status drugs were completely inactive.

Another group of rats received 50 mg/kg lacosamide followed 5 minutes later by 20 mg/kg diazepam. Full control of seizures was achieved in all rats by this combination treatment.

It is concluded that the compounds of the present invention, in particular lacosamide, or a combination of the compounds of the present invention, in particular of lacosamide, with one or more further drug used in the treatment of SE, such as benzodiazepines, anticonvulsants or barbiturates, preferably a benzodiazepine, in particular diazepam, is suitable for the treatment of refractory *status epilepticus* or for the treatment of a long-lasting SE which is or becomes refractory in the course of its duration.

### Example 3: Long term effects of lacosamide (disease-modifying effects)

SSSE was induced in rats as described in Example 1. After SSSE induction, and at least 6 months wait ("silent period") the animals were placed in EEG/telemetry/videotape continuously for two weeks for chronic EEG and video monitoring, but the second week, which was more remote from anaesthesia and surgery, was used to calculate seizure frequency (24 hours/day x 7). Electrographic seizures were captured by the Harmony software, and were confirmed by offline manual review of the EEG and videotapes. The following indices were counted: total number of spikes of seizures for 7 days of observation, mean seizure duration, light/dark distribution.

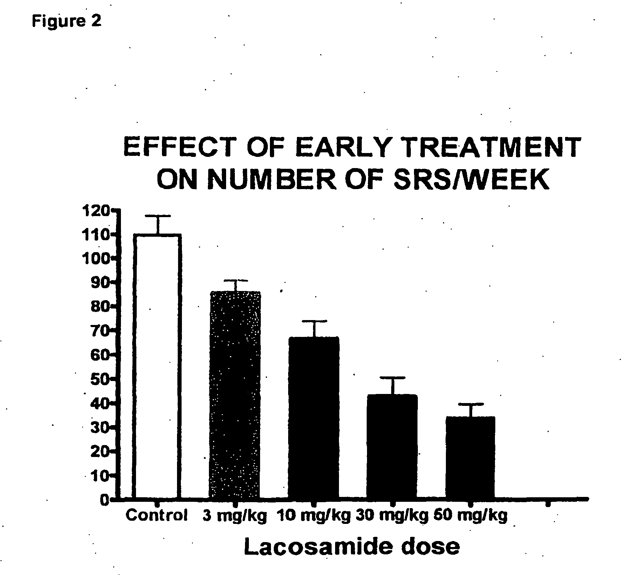

Treatment of status epilepticus 10 min after perforant path stimulation with lacosamide had significant effects on several of the long-term consequences of status epilepticus. The number of spontaneous recurrent seizures (SRSs) per week (Figure 2) was reduced from 110 ± 8 in vehicle-treated animals to 85 ± 5 in rats receiving 3 mg/kg of lacosamide, and in animals treated with 10 mg/kg, 30 mg/kg or 50 mg/kg respectively, it was 66 ± 8, 42 ± 8 and 34 ± 6.

This disease-modifying effect of small doses of lacosamide was also observed when looking at spike frequency, which was reduced from 9534 ± 1114 spikes/week in controls to 7557 ± 1945 spikes/week in the 3 mg/kg group, and to 3536 ± 380, 2969 ± 542, and 2588 ± 370 spikes/week in the 10 mg/kg, 30 mg/kg and 50 mg/kg groups, respectively.

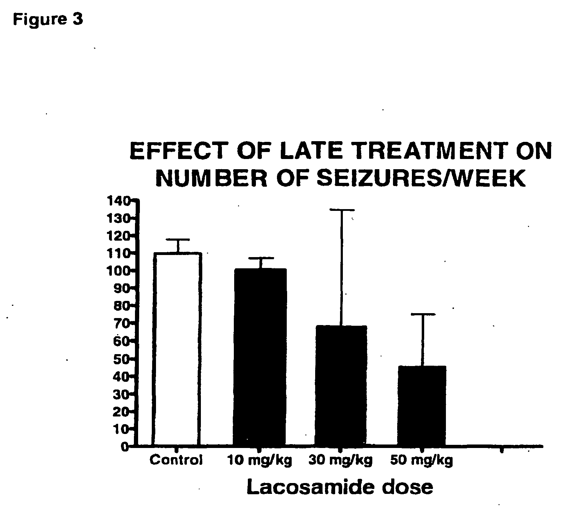

Treatment 40 min after perforant path stimulation reduced the number of animals showing spontaneous recurrent seizures from 6/6 to 3/9 in the two higher dosage treatment groups combined (p<0.05). When the two highest treatment groups were combined, they reduced seizure numbers from 110 ± 8 to 55 ± 32 seizures per week. When individual treatments were analysed, the number of seizures per week went from 110 ± 8 to 100 ± 7 (lacosamide 10 mg/kg), 67 ± 67 (lacosamide 30 mg/kg) and 45 ± 29 (lacosamide 50 mg/kg), but these changes were not statistically significant (Figure 3).

However, the median number of seizures in the 30 mg/kg and 50 mg/kg groups was 0, reflecting the fact that the majority of animals had no SRSs.

Lacosamide was effective as an anticonvulsant when given 10 min after perforant path stimulation in the development of *status epilepticus,* and at doses 10 mg/kg and above, it reduced the number of seizures, as well as the cumulative time spent seizing after treatment.

Chronically, early lacosamide treatment (10 min after perforant path stimulation) reduced the frequency of spontaneous recurrent seizures and reduce spike frequency.

Treatment of established, self-sustaining *status epilepticus* 40 min after perforant path stimulation (late treatment) produced a non-significant reduction in the number of seizures.

Treatment with lacosamide at high dose (30-50 mg/kg) reduced the incidence of chronic SRSs, and the frequency of those SRSs, suggesting a disease-modifying effect on chronic epileptogenesis.

Early treatment reduced the severity of the subsequent chronic epilepsy, a disease-modifying effect. After late treatment, a disease-modifying effect was observed when the two high-dose groups were combined for analysis.

## Claims

1. Pharmaceutical composition for use in the prevention, alleviation or/and treatment of a refractory epileptic condition, said pharmaceutical composition comprising a compound having the Formula (II) 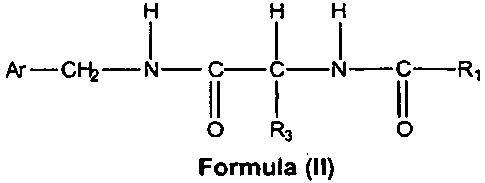 wherein
Ar is phenyl, which is unsubstituted or substituted with at least one halo group;
R₁ is alkyl containing 1 to 3 carbon atoms, and
R₃ is -CH₂-Q, wherein Q is alkoxy containing 1 to 3 carbon atoms,
or a pharmaceutically acceptable salt thereof.

2. The pharmaceutical composition for use of claim 1, wherein the refractory epileptic condition is a refractory *status epilepticus,* or/and a condition related to refractory *status epilepticus.*

3. The pharmaceutical composition for use according to claim 1 or 2, wherein R₁ is methyl.

4. The pharmaceutical composition for use according to any one of claims 1 to 3, wherein Ar is phenyl substituted with at least one fluoro group.

5. The pharmaceutical composition for use according to any one of claims 1 to 3, wherein Ar is unsubstituted phenyl.

6. The pharmaceutical composition for use according to claim 1 or 2, wherein the compound is
(R)-2-acetamido-N-benzyl-3-methoxy-propionamide;
(R)-2-acetamido-N-benzyl-3-ethoxy-propionamide;
O-methyl-N-acetyl-D-serine-m-fluorobenzylamide; or
O-methyl-N-acetyl-D-serine-p-fluorobenzylamide.

7. The pharmaceutical composition for use according to any one of the preceding claims wherein the compound is in the R configuration.

8. The pharmaceutical composition for use according to any one of the claims 1 to 7, wherein the compound of Formula (II) is (R)-2-Acetamido-N-benzyl-3-methoxypropionamide or a pharmaceutically acceptable salt thereof.

9. The pharmaceutical composition for use according to claim 8, wherein the compound is (R)-2-Acetamido-N-benzyl-3-methoxy-propionamide.

10. The pharmaceutical composition for use according to any one of the preceding claims, wherein the refractory epileptic condition is a *status epilepticus,* an epileptic seizure, a repetitive seizure or/and a seizure cluster continuing for at least about 10 min.

11. The pharmaceutical composition for use according to any one of the preceding claims, wherein the refractory epileptic condition is at least partially refractory or substantially refractory against drugs employed in the treatment of *status epilepticus* or/and epilepsy, more particular against at least one drug selected from benzodiazepines, barbiturates, and anti-epileptic drugs different from the compounds as defined in claim 1, preferably selected from diazepam, lorazepam, midazolam, phenobarbital, carbamazepine, phenytoin, fosphenytoin, oxcarbazepine, lamotrigine, gabapentin, pregabalin, valproic acid, pentobarbital, thiopental, propofol and pharmaceutically acceptable salts thereof.

12. The pharmaceutical composition for use according to any one of the preceding claims, wherein the refractory epileptic condition comprises focal seizures or/and generalized seizures and/or wherein the refractory epileptic condition comprises convulsive seizures, such as tonic-clonic, tonic, clonic, or myoclonic seizures, or/and non-convulsive seizures, such as absences or atonic seizures and/or wherein the refractory epileptic condition or a related condition comprises acute repetitive seizures or/and seizure clusters.

13. The pharmaceutical composition for use according to any one of the preceding claims, wherein the refractory epileptic condition or a related condition comprises acute repetitive seizures.

14. The pharmaceutical composition for use according to any one of the preceding claims, wherein the condition related to refractory *status epilepticus* is epileptogenesis.

15. A pharmaceutical composition comprising
(a) at least one compound of Formula (II) as defined in any one of the claims 1 and 3 to 9, preferably lacosamide, and (b) at least one benzodiazepine, preferably diazepam, lorazepam or/and midazolam.

16. Pharmaceutical composition of claim 15 for use in the prevention, alleviation or/and treatment of an epileptic disorder.

17. A pharmaceutical composition for use in the prevention, alleviation or/and treatment of epileptogenesis, said composition comprising at least one compound having the Formula (II) 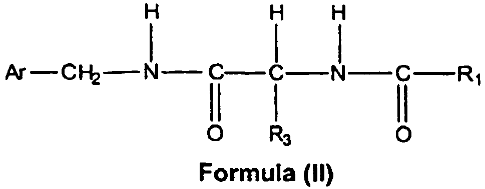 wherein
Ar is phenyl, which is unsubstituted or substituted with at least one halo group;
R₁ is alkyl containing 1 to 3 carbon atoms, and
R₃ is -CH₂-Q, wherein Q is alkoxy containing 1 to 3 carbon atoms,
or a pharmaceutically acceptable salt thereof.

18. The pharmaceutical composition for use according to claim 17, wherein epileptogenesis is related to *status epilepticus,* such as refractory *status epilepticus.*

19. The pharmaceutical composition for use according to claim 17 or 18, wherein the epileptogenesis includes the development of epilepsy, such as chronic epilepsy, or an epileptic condition selected from *status epilepticus,* an epileptic seizure, a repetitive seizure or/and a seizure cluster continuing for at least about 10 min.

20. The pharmaceutical composition for use according to claim 19, wherein the epileptic condition comprises focal seizures or/and generalized seizures and/or wherein the epileptic condition comprises convulsive seizures, such as tonic-clonic, tonic, clonic, or myoclonic seizures, or/and non-convulsive seizures, such as absences or atonic seizures or/and wherein the epileptic condition or a related condition comprises acute repetitive seizures or/and seizure clusters.

21. The pharmaceutical composition for use according to claim 19 or 20, wherein the epileptic condition or a related condition comprises acute repetitive seizures.

22. The pharmaceutical composition for use according to any one of the claims 17 to 21, wherein the compound of Formula (II) is lacosamide.

## Patentansprüche

1. Pharmazeutische Zusammensetzung zur Verwendung bei der Vorsorge, Linderung oder/und Behandlung einer refraktären epileptischen Erkrankung, wobei die pharmazeutische Zusammensetzung eine Verbindung umfasst, welche die Formel (II) aufweist 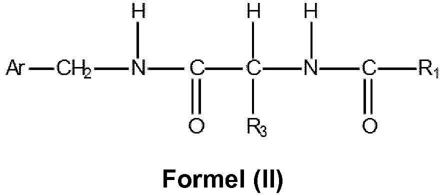 wobei
Ar Phenyl ist, welches unsubstituiert oder mit wenigstens einer Halogruppe substituiert ist,
R₁ Alkyl ist, welches 1 bis 3 Kohlenstoffatome enthält, und
R₃ -CH₂-Q ist, wobei Q Alkoxy ist, welches 1 bis 3 Kohlenstoffatome enthält,
oder ein pharmazeutisch akzeptables Salz davon.

2. Pharmazeutische Zusammensetzung zur Verwendung gemäß Anspruch 1, wobei die refraktäre epileptische Erkrankung ein refraktärer *Status epilepticus* oder/und eine Erkrankung ist, welche mit refraktärem *Status epilepticus* zusammenhängt.

3. Pharmazeutische Zusammensetzung zur Verwendung gemäß Anspruch 1 oder 2, wobei R₁ Methyl ist.

4. Pharmazeutische Zusammensetzung zur Verwendung gemäß einem der Ansprüche 1 bis 3, wobei Ar Phenyl ist, welches mit wenigstens einer Fluorgruppe substituiert ist.

5. Pharmazeutische Zusammensetzung zur Verwendung gemäß einem der Ansprüche 1 bis 3, wobei Ar unsubstituiertes Phenyl ist.

6. Pharmazeutische Zusammensetzung zur Verwendung gemäß Anspruch 1 oder 2, wobei die Verbindung
(R)-2-Acetamido-N-benzyl-3-methoxypropionamid,
(R)-2-Acetamido-N-benzyl-3-ethoxypropionamid,
O-Methyl-N-acetyl-D-serin-m-fluorbenzylamid, oder
O-Methyl-N-acetyl-D-serin-p-fluorbenzylamid ist.

7. Pharmazeutische Zusammensetzung zur Verwendung gemäß einem der vorhergehenden Ansprüche, wobei die Verbindung in der R-Konfiguration vorliegt.

8. Pharmazeutische Zusammensetzung zur Verwendung gemäß einem der Ansprüche 1 bis 7, wobei die Verbindung der Formel (II) (R)-2-Acetamido-N-benzyl-3-methoxypropionamid oder ein pharmazeutisch akzeptables Salz davon ist.

9. Pharmazeutische Zusammensetzung zur Verwendung gemäß Anspruch 8, wobei die Verbindung (R)-2-Acetamido-N-benzyl-3-methoxypropionamid ist.

10. Pharmazeutische Zusammensetzung zur Verwendung gemäß einem der vorhergehenden Ansprüche, wobei die refraktäre epileptische Erkrankung ein *Status epilepticus,* ein epileptischer Anfall, ein repetitiver Anfall oder/und ein Anfallscluster ist, welcher sich für wenigstens etwa 10 min fortsetzt.

11. Pharmazeutische Zusammensetzung zur Verwendung gemäß einem der vorhergehenden Ansprüche, wobei die refraktäre epileptische Erkrankung wenigstens teilweise refraktär oder im Wesentlichen refraktär gegen Wirkstoffe ist, welche bei der Behandlung von *Status epilepticus* oder/und Epilepsie verwendet werden, insbesondere gegen wenigstens einen Wirkstoff ausgewählt aus Benzodiazepinen, Barbituraten und antiepileptischen Wirkstoffen, welche von den Verbindungen wie in Anspruch 1 definiert verschieden sind, vorzugsweise ausgewählt aus Diazepam, Lorazepam, Midazolam, Phenobarbital, Carbamazepin, Phenytoin, Fosphenytoin, Oxcarbazepin, Lamotrigin, Gabapentin, Pregabalin, Valproinsäure, Pentobarbital, Thiopental, Propofol und pharmazeutisch akzeptablen Salzen davon.

12. Pharmazeutische Zusammensetzung zur Verwendung gemäß einem der vorhergehenden Ansprüche, wobei die refraktäre epileptische Erkrankung fokale Anfälle oder/und generalisierte Anfälle umfasst, und/oder wobei die refraktäre epileptische Erkrankung konvulsive Anfälle, wie zum Beispiel tonisch-klonische, tonische, klonische oder myoklonische Anfälle, oder/und nicht-konvulsive Anfälle umfasst, wie zum Beispiel Absencen oder atonische Anfälle, und/oder wobei die refraktäre epileptische Erkrankung oder eine damit zusammenhängende Erkrankung akute repetitive Anfälle oder/und Anfallscluster umfasst.

13. Pharmazeutische Zusammensetzung zur Verwendung gemäß einem der vorhergehenden Ansprüche, wobei die refraktäre epileptische Erkrankung oder eine damit zusammenhängende Erkrankung akute repetitive Anfälle umfasst.

14. Pharmazeutische Zusammensetzung zur Verwendung gemäß einem der vorhergehenden Ansprüche, wobei die Erkrankung, welche mit refraktärem *Status epilepticus* zusammenhängt, Epileptogenese ist.

15. Pharmazeutische Zusammensetzung umfassend
(a) wenigstens eine Verbindung der Formel (II) wie in einem der Ansprüche 1 und 3 bis 9 definiert, vorzugsweise Lacosamid, und (b) wenigstens ein Benzodiazepin, vorzugsweise Diazepam, Lorazepam oder/und Midazolam.

16. Pharmazeutische Zusammensetzung gemäß Anspruch 15 zur Verwendung bei der Vorsorge, Linderung oder/und Behandlung einer epileptischen Erkrankung.

17. Pharmazeutische Zusammensetzung zur Verwendung bei der Vorsorge, Linderung oder/und Behandlung von Epileptogenese, wobei die pharmazeutische Zusammensetzung wenigstens eine Verbindung umfasst, welche die Formel (II) aufweist 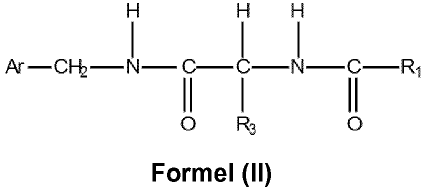 wobei
Ar Phenyl ist, welches unsubstituiert oder mit wenigstens einer Halogruppe substituiert ist,
R₁ Alkyl ist, welches 1 bis 3 Kohlenstoffatome enthält, und
R₃ -CH₂-Q ist, wobei Q Alkoxy ist, welches 1 bis 3 Kohlenstoffatome enthält,
oder ein pharmazeutisch akzeptables Salz davon.

18. Pharmazeutische Zusammensetzung zur Verwendung gemäß Anspruch 17, wobei die Epileptogenese mit *Status epilepticus* zusammenhängt, wie zum Beispiel refraktärem *Status epilepticus.*

19. Pharmazeutische Zusammensetzung zur Verwendung gemäß Anspruch 17 oder 18, wobei die Epileptogenese die Entwicklung von Epilepsie einschließt, wie zum Beispiel einer chronischen Epilepsie oder einer epileptischen Erkrankung, welche ausgewählt ist aus *Status epilepticus,* einem epileptischen Anfall, einem repetitiver Anfall oder/und einem Anfallscluster, welcher sich für wenigstens etwa 10 min fortsetzt.

20. Pharmazeutische Zusammensetzung zur Verwendung gemäß Anspruch 19, wobei die epileptische Erkrankung fokale Anfälle oder/und generalisierte Anfälle umfasst, und/oder wobei die epileptische Erkrankung konvulsive Anfälle, wie zum Beispiel tonisch-klonische, tonische, klonische oder myoklonische Anfälle, oder/und nicht-konvulsive Anfälle umfasst, wie zum Beispiel Absencen oder atonische Anfälle, oder/und wobei die epileptische Erkrankung oder eine damit zusammenhängende Erkrankung akute repetitive Anfälle oder/und Anfallscluster umfasst.

21. Pharmazeutische Zusammensetzung zur Verwendung gemäß Anspruch 19 oder 20, wobei die epileptische Erkrankung oder eine damit zusammenhängende Erkrankung akute repetitive Anfälle umfasst.

22. Pharmazeutische Zusammensetzung zur Verwendung gemäß einem der Ansprüche 17 bis 21, wobei die Verbindung der Formel (II) Lacosamid ist.

## Revendications

1. Composition pharmaceutique pour l'utilisation dans la prévention, le soulagement ou/et le traitement d'une affection épileptique réfractaire, ladite composition pharmaceutique comprenant un composé ayant la Formule (II) 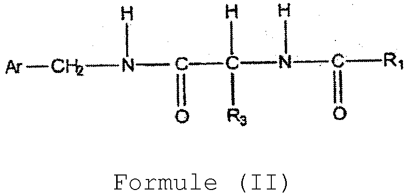 dans laquelle
Ar est un phényle, qui est non substitué ou substitué par au moins un groupe halogéno ;
R₁ est un alkyle contenant de 1 à 3 atomes de carbone, et
R₃ est -CH₂-Q, où Q est un alcoxy contenant de 1 à 3 atomes de carbone,
ou un sel pharmaceutiquement acceptable de celui-ci.

2. Composition pharmaceutique pour l'utilisation selon la revendication 1, dans laquelle l'affection épileptique réfractaire est un état de mal épileptique réfractaire, ou/et une affection apparentée à un état de mal épileptique réfractaire.

3. Composition pharmaceutique pour l'utilisation selon la revendication 1 ou 2, dans laquelle R₁ est un méthyle.

4. Composition pharmaceutique pour l'utilisation selon l'une quelconque des revendications 1 à 3, dans laquelle Ar est un phényle substitué par au moins un groupe fluoro.

5. Composition pharmaceutique pour l'utilisation selon l'une quelconque des revendications 1 à 3, dans laquelle Ar est un phényle non substitué.

6. Composition pharmaceutique pour l'utilisation selon la revendication 1 ou 2, dans laquelle le composé est
le (R)-2-acétamido-N-benzyl-3-méthoxy-propionamide ;
le (R)-2-acétamido-N-benzyl-3-éthoxy-propionamide ;
le O-méthyl-N-acétyl-D-sérine-m-fluorobenzylamide ; ou
le O-méthyl-N-acétyl-D-sérine-p-fluorobenzylamide.

7. Composition pharmaceutique pour l'utilisation selon l'une quelconque des revendications précédentes, dans laquelle le composé est dans la configuration R.

8. Composition pharmaceutique pour l'utilisation selon l'une quelconque des revendications 1 à 7, dans laquelle le composé de Formule (II) est le (R)-2-acétamido-N-benzyl-3-méthoxy-propionamide ou un sel pharmaceutiquement acceptable de celui-ci.

9. Composition pharmaceutique pour l'utilisation selon la revendication 8, dans laquelle le composé est le (R)-2-acétamido-N-benzyl-3-méthoxy-propionamide.

10. Composition pharmaceutique pour l'utilisation selon l'une quelconque des revendications précédentes, dans laquelle l'affection épileptique réfractaire est un état de mal épileptique, une crise d'épilepsie, une répétition de crises d'épilepsie ou/et un épisode épileptique se poursuivant pendant au moins environ 10 min.

11. Composition pharmaceutique pour l'utilisation selon l'une quelconque des revendications précédentes, dans laquelle l'affection épileptique réfractaire est au moins partiellement réfractaire ou essentiellement réfractaire aux substances médicamenteuses employées dans le traitement d'un état de mal épileptique ou/et d'une épilepsie, plus particulièrement à au moins une substance médicamenteuse choisie parmi les benzodiazépines, les barbituriques, et les substances médicamenteuses antiépileptiques différentes des composés tels que définis dans la revendication 1, de préférence choisie parmi le diazépam, le lorazépam, le midazolam, le phénobarbital, la carbamazépine, la phénytoïne, la fosphénytoïne, l'oxcarbazépine, la lamotrigine, la gabapentine, la prégabaline, l'acide valproïque, le pentobarbital, le thiopental, le propofol et les sels pharmaceutiquement acceptables de ceux-ci.

12. Composition pharmaceutique pour l'utilisation selon l'une quelconque des revendications précédentes, dans laquelle l'affection épileptique réfractaire comprend des crises focales ou/et des crises généralisées et/ou dans laquelle l'affection épileptique réfractaire comprend des crises convulsives, telles que des crises tonico-cloniques, toniques, cloniques ou myocloniques, ou/et des crises non convulsives, comme des absences ou des crises atoniques et/ou dans laquelle l'affection épileptique réfractaire ou une affection apparentée comprend des répétitions de crises aiguës ou/et des épisodes épileptiques.

13. Composition pharmaceutique pour l'utilisation selon l'une quelconque des revendications précédentes, dans laquelle l'affection épileptique réfractaire ou une affection apparentée comprend des répétitions de crises aiguës.

14. Composition pharmaceutique pour l'utilisation selon l'une quelconque des revendications précédentes, dans laquelle l'affection apparentée à un état de mal épileptique réfractaire est une épileptogenèse.

15. Composition pharmaceutique comprenant
(a) au moins un composé de Formule (II) tel que défini dans l'une quelconque des revendications 1 et 3 à 9, de préférence le lacosamide, et (b) au moins une benzodiazépine, de préférence le diazépam, le lorazépam ou/et le midazolam.

16. Composition pharmaceutique selon la revendication 15, pour l'utilisation dans la prévention, le soulagement ou/et le traitement d'un trouble épileptique.

17. Composition pharmaceutique pour l'utilisation dans la prévention, le soulagement ou/et le traitement d'une épileptogenèse, ladite composition comprenant au moins un composé ayant la Formule (II) 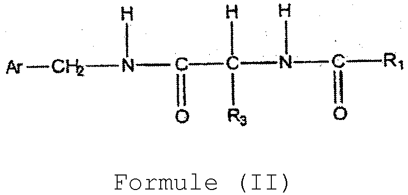 dans laquelle
Ar est un phényle, qui est non substitué ou substitué par au moins un groupe halogéno ;
R₁ est un alkyle contenant de 1 à 3 atomes de carbone, et
R₃ est -CH₂-Q, où Q est un alcoxy contenant de 1 à 3 atomes de carbone,
ou un sel pharmaceutiquement acceptable de celui-ci.

18. Composition pharmaceutique pour l'utilisation selon la revendication 17, dans laquelle l'épileptogenèse est apparentée à un état de mal épileptique, tel qu'un état de mal épileptique réfractaire.

19. Composition pharmaceutique pour l'utilisation selon la revendication 17 ou 18, dans laquelle l'épileptogenèse inclut le développement d'une épilepsie, telle qu'une épilepsie chronique, ou une affection épileptique choisie parmi un état de mal épileptique, une crise d'épilepsie, une répétition de crises d'épilepsie ou/et un épisode épileptique se poursuivant pendant au moins environ 10 min.

20. Composition pharmaceutique pour l'utilisation selon la revendication 19, dans laquelle l'affection épileptique comprend des crises focales ou/et des crises généralisées et/ou dans laquelle l'affection épileptique comprend des crises convulsives, telles que des crises tonico-cloniques, toniques, cloniques ou myocloniques, ou/et des crises non convulsives, comme des absences ou des crises atoniques ou/et dans laquelle l'affection épileptique ou une affection apparentée comprend des répétitions de crises aiguës ou/et des épisodes épileptiques.

21. Composition pharmaceutique pour l'utilisation selon la revendication 19 ou 20, dans laquelle l'affection épileptique ou une affection apparentée comprend des répétitions de crises aiguës.

22. Composition pharmaceutique pour l'utilisation selon l'une quelconque des revendications 17 à 21, dans laquelle le composé de Formule (II) est le lacosamide.
